# EUROPEAN PATENT APPLICATION

(11) **EP 2 716 315 A1**
(43) Date of publication of application: **09.04.2014**
(21) Application number: 12187583.5
(22) Date of filing: 08.10.2012
(51) Int. Cl.: A61M 5/168, A61M 5/142, A61M 5/145, A61M 5/31, A61M 5/315

(54) **Precision liquid dispensing device**

(71) Applicant: IN. Medica, d.o.o., 8310 Sentjernej (SI)
(72) Inventor: Pavlin, Marko, 8000 Novo Mesto (SI)
(74) Representative: Ivancic, Bojan

(57) **Abstract**

The present invention refers to a precision liquid dispensing device, in particular to a portable disposable device for controlled medication dispensing during certain time period, such as insulin for instance and similar, comprising a body with a container to accept liquid and a movable piston to press said liquid out of said container. It is provided for, according to the present invention, that cavity (6) is formed in a body (1) communicating at its first end via an aperture in a wall (3) of the body (1) with the surrounding. A movable piston (7) is arranged in said cavity (6) with the outline being complementary with the outline of the cavity (6). Said piston (7) is formed with a through-hole (8) extending approximately in parallel with the course of said cavity (6).

## Description

The present invention refers to a precision liquid dispensing device, in particular to a portable disposable device for controlled medication dispensing during certain time period, such as insulin for instance and similar, comprising a body with a container to accept liquid and a movable piston to press said liquid out of said container.

Devices as mentioned in the foregoing are known and comprise, in general, a longitudinal cylindrical container wherefrom liquid, such as insulin for instance, being dispensed by means of a piston inserted into said container. A drawback of the known devices is represented by a relative large length thereof, since initially, with the filled container, said piston with a piston rod is retracted from the container which in turn represents a double length of the device. Due to this fact said known devices are rather unhandy and user-unfriendly.

It is the object of the present invention to create a precision liquid dispensing device, in particular to a portable disposable device for controlled medication dispensing during certain time period, such as insulin for instance and similar, which remedies drawbacks of the known solutions.

The object as set above is solved by characteristics set forth in a characterising clause of the claim 1.

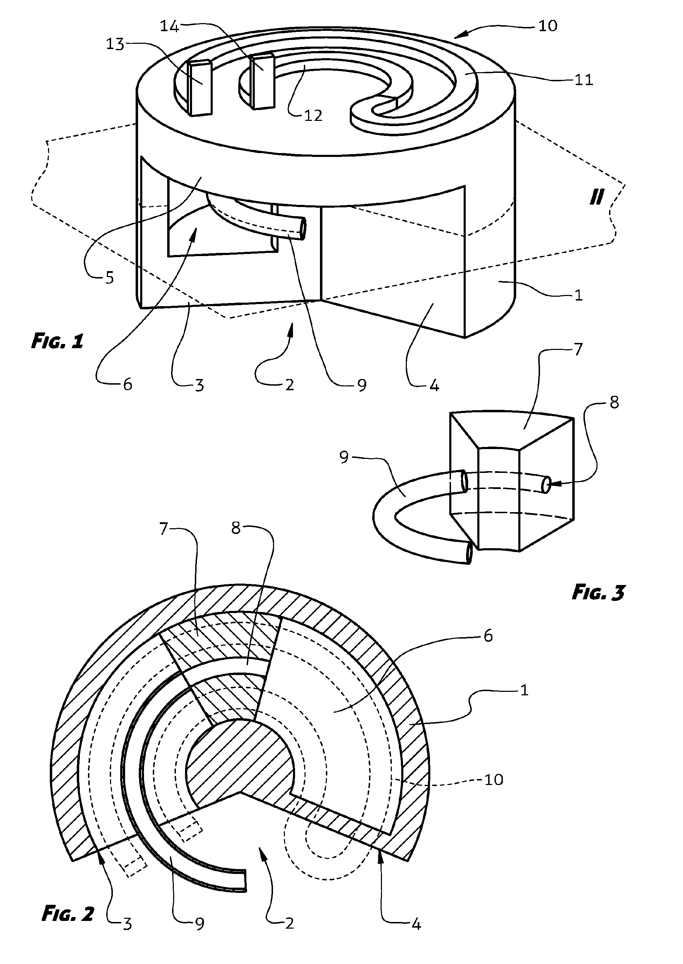

The invention is further described in detail by way of non-limiting preferred embodiment, and with a reference to the accompanying drawing, where
- Fig. 1: shows a three-dimensional view of a device according to the invention,
- Fig. 2: shows a cross-sectional view of a device of Fig. 1 through a horizontal plane **II** extending through a centre-line of a container with a pressure unit, and
- Fig. 3: shows a three-dimensional view of a pressure unit of a device of Fig. 1.

A precision liquid dispensing device according to the present invention comprises a compact body, i.e. a cylindrical body 1 in the present embodiment, being formed at the side of the first base thereof, i.e. at the bottom base in the present embodiment, with a recess 2 delimited by vertical walls 3, 4 and a top section 5. A longitudinally intensive cavity 6 is formed inside said body 1 serving as a container to accept the liquid to be dispensed by the device according to the invention. Said cavity 6 is terminated at the first end thereof by means of said wall 4, whereas it penetrates said wall 3 at the second end thereof. A piston 7 moveable in the course of the cavity 6 is arranged in said cavity, the outline of said piston 7 being complementary to the outline of the cavity 6. Said piston 7 is formed with a through-hole 8 extending approximately in parallel with the course of said cavity 6. A tubular projection 9 is attached to the side of the piston 7 facing the aperture in said wall 3, said tubular projection being intended for the attachment of a conduit (not shown in the Fig.) for inflow and outflow the liquid to be dispensed by the device according to the invention. According to the present invention, said tubular projection 9 is formed as a relatively flexible tubular projection extending over said cavity 6 from said piston 7 to the aperture area in said wall 3. Said cavity 6 is formed, according to the present invention, as a cut-out created by a solid of revolution of an arbitrary shape, preferably a circle, a rectangle and similar, said shape being rotated for an appropriate angle about the centre-line of said body 1, where said centre-line lies on the same plane and more distant from the centre of said shape as the biggest dimension thereof.

On the surface of said top section 5 above said cavity 6 is arranged a metering means 10, in a potentiometer in the shown embodiment, the form thereof being adapted to the course of said cavity 6. Said metering means 10 is formed of two sections 11, 12 running in parallel, the first section being resistive and the second conductive. Said sections 11, 12 are mutually linked at each first end, the free ends thereof being provided with sliding contacts 13,14.

In initial position of the device according to the invention, when the cavity 6 is still empty, is the piston 7 located at the aperture of the wall 3 of the body whereas the tubular projection 9 is located entirely outside the cavity 6. A pumping means forwarding the liquid from the cavity 6 is attached to said tubular projection 9 via additional conduit, however, such an attachment is known per se and, thus, neither described nor shown in detail in the drawing. The entire system comprising everything from said cavity 6, tubular projection 9, said conduit and the pumping means, is filled with the liquid to be dispensed by device according to the invention. Afterwards, in a certain moment, the device according to the invention is activated, which is followed by forwarding by means of said pumping means the liquid to be dispensed via said additional conduit, tubular projection 9 and through hole 8 in the piston 7 out of the cavity 6. The suction of the liquid to be dispensed creates a low pressure in the cavity 6 resulting in the surrounding high pressure to act upon the free surface of the piston 7 being in connection via said aperture in the wall 3 with the surrounding. As a result, said piston 7 advances in the cavity 6 until the terminal wall 4 is reached when the entire liquid to be dispensed has been pumped out, and the device according to the invention stops.

The amount of pumped-out liquid to be dispensed is metered at the pumping means in a manner known per se. Additionally, the amount of the pumped-out liquid to be dispensed is metered by means of said metering means 10. Said metering means senses the momentary position of the head of the piston 7 with regard to the terminal wall 4, and based on that results the device according to the invention calculates the amount of the liquid to be dispensed remained in the cavity 6 and the amount of the liquid already pumped out, respectively. Comparing the dispensed amount of the liquid obtained directly at the pumping means with the amount of the liquid obtained by means of said metering means 10 on the body 1 of the device according to the invention, it can be very simple to determine the exact amount of the pumped-out liquid to be dispensed. In such a manner, it can be efficiently and reliably prevented to overdose and under-dose the liquid to be dispensed by means of the device according to the present invention.

The embodiment represented in the drawing provides for that the piston 7 resembles in the cross-section a form of a square, and that the trajectory of the cavity 6 resembles the form of a planar section of a circular ring. However, it is obvious for the person skilled in the art that the piston 7 may resemble other cross-sectional forms such as a circle and similar, and that said trajectory of the cavity 6 may resemble other forms without departing from the spirit and scope of the invention.

## Claims

1. A precision liquid dispensing device, in particular a portable disposable device for controlled medication dispensing during certain time period, such as insulin for instance and similar, comprising a body with a container to accept liquid and a movable piston to press said liquid out of said container, ***characterized in that*** a cavity (6) is formed in a body (1) communicating at its first end via an aperture in a wall (3) of the body (1) with the surrounding, a movable piston (7) being arranged in said cavity (6) with the outline being complementary with the outline of the cavity (6), said piston (7) being formed with a through-hole (8) extending approximately in parallel with the course of said cavity (6), to the side of the piston (7) facing the aperture in said wall (3) there is attached a tubular projection (9) intended for inflow and outflow the liquid to be dispensed, and on the surface of a top section (5) above said cavity (6) is arranged a metering means (10) for metering the amount of pumped-out liquid to be dispensed, where the form of said metering means (10) being adapted to the course of said cavity (6).

2. A precision liquid dispensing device according to claim 1, ***characterized in that*** said cavity (6) is formed as a cut-out created by a solid of revolution of an arbitrary shape rotated for an appropriate angle about the centre-line of said body (1), where said centre-line lies on the same plane and more distant from the centre of said shape as the biggest dimension thereof.

3. A precision liquid dispensing device according to claim 2, ***characterized in that*** said shape is selected as a circle, a rectangle and similar.

4. A precision liquid dispensing device according to claim 1, ***characterized in that*** said tubular projection (9) is formed as a relatively flexible tubular projection.
